# EUROPEAN PATENT SPECIFICATION

(11) **EP 2 676 555 B1**
(45) Date of publication and mention of the grant of the patent: **25.12.2019**
(21) Application number: 12747509.3
(22) Date of filing: 16.02.2012
(51) Int. Cl.: A23L 29/20, A23L 33/21, A61K 36/185, A23L 33/24, A23L 33/28

(54) **HIGH-MOLECULAR-WEIGHT POLYMER FOR USE**
POLYMERS MIT HOHEM MOLEKULARGEWICHT FÜR VERWENDUNG
POLYMÈRE DE MASSE MOLÉCULAIRE ÉLEVÉE POUR UTILISATION

(30) Priority: 16.02.2011 CN 201110039278
(43) Date of publication of application: 25.12.2013
(73) Proprietor: Hsin, Shaochi, Taipei County 242 (TW)
(72) Inventor: Hsin, Shaochi, Taipei County 242 (TW)
(74) Representative: Körfer, Thomas
(86) International application number: PCT/CN2012/071184
(87) International publication number: WO 2012/109991

(56) References cited:
- WO-A2-02/40039
- WO-A2-2009/027985
- WO-A2-2009/100181
- CN-A- 1 308 899
- CN-A- 1 806 700
- CN-U- 201 710 674
- US-A1- 2008 095 881
- Anonymous: "Normacol Plus - Summary of Product Characteristics (SmPC) - (eMC)", , 1 January 2009 (2009-01-01), XP055595753, Retrieved from the Internet: URL:https://www.medicines.org.uk/emc/produ ct/1027/smpc [retrieved on 2019-06-12]

## Description

### FIELD OF THE INVENTION

The present invention relates to a high molecular weight polymer, especially the invention relates to a high molecular weight polymer for use in indirectly adsorbing or absorbing harmful or toxic substances existed or contained in body fluid or solution so that they can be removed, and a composition containing a high molecular weight polymer.

### BACKGROUND OF THE INVENTION

A conventional high molecular weight polymer includes, for example, a natural biomedical polymeric material (such as a high molecular weight fiber or a polymer which is originated from animal including a collagen, a gelatin, a hyaluronic acid, a chitin, a chitosan and a derivative thereof and a high molecular weight fiber or polymer which is originated from plant including an alginates, a cellulose and a derivative thereof. Since it has excellent biocompatibility and bio-degradability and contains hydrolysable bonding, it can be degraded to small molecules in an organism, and is removed out of the body through a kidney filtering and metabolizing.

Those high molecular weight fibers and polymers might be used as a food additive due to well swelling property, so they can generate full sense after eating, and decrease dietary requirement to achieve the purpose of slimming. Moreover, the high molecular weight polymers comprise a lot of fiber so that they can improve the movement of the gastrointestinal tract and be used as a stool softener (such as Sterculia BP or Frangula BPC) to promote the defecation of a constipated patient.

However, the above mentioned applications of the high molecular weight fibers and polymers focus on their superior water adsorbability but other potential applications are ignored. Therefore, their actually industrial values are decreased.

WO 2009/027985 A2 describes the treatment of gastrointestinal tract using fiber/granule complex. In contrast to the present invention, the fiber of this document is non-absorbing in nature and the harmful substance to be removed are fixed to the granules (not the fiber). Therefore, the fiber of this document merely acts as a carrier for the active composition. In contrary, the high molecular fiber of the present invention absorbs toxins and removes it from the body. Further, this document completely fails to disclose Sterculia BP or Frangula BPC as high molecular weight polymer.

WO 02/40039 A2 describes to a method to treat a serious medical condition of fluid overload. This document discloses methods and materials for removal of fluid from the intestinal tract of the host. The enteric coating releases the polymer when it reaches the specific location wherein the water and the toxins are absorbed.

WO 2009/100181 A2 describes a composition containing a high molecular weight polymer such as cellulose and an excipient, a composition suitable for oral administration for using absorbing toxins such as alcohol and remove from the body. This document focuses on appetite suppression and weight management achieved through pre-meal bulking agent. Therefore, this document is directed to appetite suppression and weight management whereas the present invention relates to absorbing toxins.

US 2008/0095881 A1 teaches detoxification to remove toxins accumulated over a period in the body, especially in the liver, kidney, skin and other organs. For example, it teaches Chelators to bind with metal ions and other toxins in the body to aid in detoxification of a subject. Therefore, the composition of this document merely aims to upkeep the general health and remove toxin that builds up over time. In the case of the present invention, toxins relate to substances the can cause immediate impairment when ingested by a subject. The present invention aims at removal of such toxins before the body absorbs it, which can cause impairment or damage. Therefore, a person skilled in the art would not be motivated to consult this document, which aims at detoxification in the mere sense of general health up keeping. Further, this document does not deal with toxins that can cause immediate damage to the subject but that accumulates over time. The present invention recites Sterculia BP and Frangula BPC, which are natural fiber capable of absorbing harmful substance from the human body.

CN 201 710 674 U teaches an adsorption isolating device with a core surrounded by an outer shell. The core layer is a macromolecular polymeric material such as cellulose, colloid, carbohydrate and the shell layer is made of an inactive material such as activated carbon.

### DESCRIPTIONS OF THE INVENTION

The object of the invention is to provide a high molecular weight polymer for use in the absorption of body fluid (such as digestive juice) in an organism or of a liquid or solution to be intaked into an organism containing a toxin or a stimulating component. That relates to direct absorption of a liquid or solution to indirectly adsorb the toxins or stimulating components existed therein, decrease of digestive juice in body, decrease of digestion and absorption of food in a body and improvement of excretion of these harmful substances to prevent the body from being damaged by the toxin or the stimulating substance. These high molecular weight polymers have good biocompatibility, so they will not harm health of an organism after being intaked into it.

The invention applies a high molecular weight polymer to a body fluid (such as digestive juice) in an organism or a liquid or a solution to be intaked into an organism so that harmful or toxic substances contained therein can be removed through indirect adsorption or absorption and excretion. Since most high molecular weight polymers have a common feature in adsorbing liquid, they can indirectly absorb the harmful or toxic substances in the liquid. Therefore, the application field of the high-molecular weight polymer is wider and the polymer has an improved commercial potential.

The high-molecular weight polymer of the invention is a natural fiber which is Sterculia BP or Frangula BPC. These natural fibers have high cross-linking molecular structure, so they can absorb alcohol in wine and a liquid or solution that may have harmful substance (such as caffeine, theine, uric acid, lipid, cholesterol, strong acid and strong base) by utilizing the feature of directly absorption of the liquid or solution to indirect adsorb toxic substances, and then they are excreted from the body to reduce absorption of such substances by the body, so the toxicity, side effect and adverse effect caused by such substances can be further reduced to achieve the effect of protecting organ, tissue and cells of an organism.

The high molecular weight polymer can be combined with a layer of biocompatible excipient, for example, an outer shell formed by the excipient, which has a plurality of micropores. When the high molecular weight polymer is taken by an organism, the biocompatible excipient can prevent the high molecular weight polymer from damage by a body fluid of the organism, an acid or a base. After the high molecular weight polymer coated with the biocompatible excipient is orally administered to an organism, harmful substances in the organism enter into the composition through the micropores of the biocompatible excipient and then absorbed by the high molecular weight polymer. Since the high molecular weight polymer has a cross-linking structure, so micropores can be constituted. Thus, harmful substances can be fixed in the micropores to reduce contact of the substances with tissues of the organism and prevent the organism from damage by these harmful substances.

Since absorption of a liquid is a common feature of most high molecular weight polymers, they can absorb body fluid (such as digestive juice) in an organism to decrease the digestive juice in body and thus reduce digestion and absorption of a food. Alternatively, they can assist to adsorb harmful substances contained in body fluid of a body and remove the harmful substances. Therefore, the application field of the high-molecular weight polymer is wider and the polymer has an improved commercial potential.

### BRIEF DESCRIPTION OF THE DRAWINGS

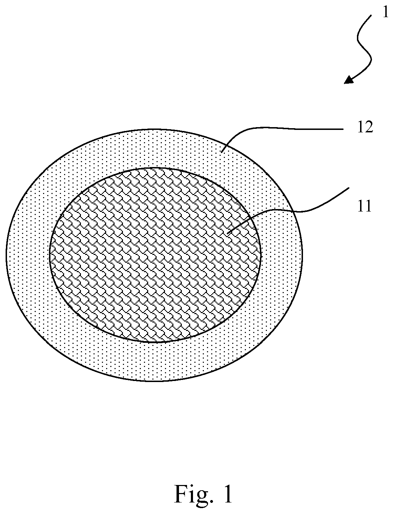

Fig. 1 is a cross- sectional view of a composition comprising a high molecular weight polymer in accordance with the present invention.

### DESCRIPTIONS OF THE SYMBOLS OF THE ELEMENTS

- 1: Composition comprising a high molecular weight polymer
- 11: Layer of high molecular weight polymer
- 12: Layer of outer shell

### PREFERRED EMBODIMENTS OF THE INVENTION

The invention provides a high molecular polymer to adsorb a body fluid (such as digestive juice) or a liquid or solution in an organism containing toxins or stimulating components (by directly absorb the liquid or solution to indirectly adsorb toxic substances) to decrease body fluid in a body, reduce digestion and absorption of food by a body or prevent tissues of an organism from direct contact of these harmful substances, absorption of the harmful substances and hazard to health. Since absorption of a liquid is a common feature of most high molecular weight polymers, by utilizing the common feature of the high molecular weight polymer in absorbing a liquid, the harmful substances contained in the liquid can be indirectly adsorb and then excreted. Therefore, the application field of the high-molecular weight polymeric composition is wider and the polymer has an improved commercial potential.

The high-molecular weight polymer of the invention is a natural fiber selected from the group consisting of: Sterculia BP and Frangula BPC. These natural fibers have good biocompatible and network molecular structure with slits in various sizes. Therefore, after the high molecular weight polymer coated with the biocompatible excipient is taken by an organism, no hazard can be caused. In addition, harmful substances can be fixed in the slits of the high molecular weight polymer to prevent the organism from contact with the harmful substances and hazard by these harmful substances.

### Example 1

In this example, Sterculia BP is selected to adsorb a coffee solution to illustrate the high molecular weight polymer for use of the present invention. The Sterculia BP is a kind of high molecular fiber, which can be applied single or mixed with another adsorptive polymer material. In this example, 25% by weight of Sterculia BP is mixed with 75% by weight of Frangula BPC to from a high molecular weight fiber mixture (hereafter referred to as "high molecular weight fiber"). One gram of the high molecular weight fiber mixture is added into 30 ml espresso coffee and then mixed well to form a fiber-coffee mixture. After 5 minutes, the fiber-coffee mixture exhibited sticky. After 10 minutes, the fiber-coffee mixture coverts to a semisolid colloid and was continuously getting sticky. After 30 minutes, the fiber-coffee mixture was almost solidified and lost fluidity. It can be known from this example that when a person intaked the fiber mixture before drinking the espresso coffee, the caffeine in the coffee can be adsorbed by the fiber-coffee mixture through the adsorption of espresso coffee. Therefore, most caffeine is isolated from the cells of human body so that the damage to health resulting from the caffeine can be reduced.

The Sterculia BP and Frangula BPC can be mixed with any ratio. The Sterculia BP and Frangula BPC can be used individually or be mixed with any other high molecular weight polymer.

### Example 2

A cola that is commonly consumed by general public was used as an example to illustrate the high molecular weight fiber for use of the present invention. The 25% by weight of Sterculia BP is mixed with 75% by weight of Frangula BPC to from a high molecular weight fiber mixture. One gram of fiber mixture is added into 30 ml cola then mixed well to form a high molecular fiber-cola mixture. After 5 minutes, the high molecular fiber-coffee mixture exhibited sticky. After 10 minutes, the viscosity of the fiber-cola mixture increased and around 95% cola was absorbed by the fiber-cola mixture and lost fluidity. After 30 minutes, the fiber-cola mixture was almost solidified. Obviously, the high molecular weight fiber can rapidly absorb the liquid in a short time. During the absorption, toxic or harmful substances can be adsorbed by the fiber accordingly to achieve the effect of preventing body from contact with and damage by these harmful substances.

### Example 3

This example illustrates the effect of absorbing alcohol by the high molecular weight polymer of Example 1. One gram high molecular weight polymer mixture was added into 30 ml sorghum wine containing 58% alcohol to form a high molecular weight fiber- sorghum wine mixture. The high molecular weight fiber-wine mixture exhibited stringy after 5 minutes mixing. After 10 minutes, the high molecular weight fiber-wine mixture showed gel exhibition. After 20∼30 minutes, the high molecular weight fiber-wine mixture showed paste exhibition. After one hour, the high molecular weight fiber-wine mixture lost fluidity and became solid state. Accordingly, the high-molecular weight polymer of the present invention has capability to adsorb the intaked alcohol and prevent the alcohol from directly contacting the tissue and generating health damage.

One gram Sterculia BP was added to 50 ml sorghum wine and Sterculia BP exhibited absorption similar to the above fiber and the high molecular weight fiber-sorghum wine mixture gradually exhibited semi-fluidity state or non-fluid paste or solid state. The high molecular weight fiber was mixed with sorghum wine containing 58% alcohol and then stirred to form a high molecular weight fiber-wine mixture. After 5 minutes, the high molecular weight fiber-wine mixture exhibited sticky. After 10 minutes, the high molecular weight fiber-wine mixture showed gel exhibition. After 20∼30 minutes, the high molecular weight fiber-wine mixture showed paste exhibition. After one hour, the high molecular weight fiber-wine mixture lost fluidity and became solid state. Accordingly, the high-molecular weight polymer of the present invention has capability to adsorb the intaked alcohol and prevent the alcohol from directly contacting the tissue and generating health damage.

The high molecular weight polymer can be produced in a chewable form and consumers can be administered by chewing; especially for those consumers who need to drink wine frequently. If the high molecular weight polymer is intaked before drinking, upon drinking, the high molecular weight polymer stayed in gastrointestinal tract can absorb alcohol in the wine so that not too much alcohol is absorbed by body through cells in the gastrointestinal tract and the body will not be harmed by alcohol. The high molecular weight polymer also can be applied in patients dependent on stimulating components such as caffeine and theine. The high molecular weight polymer of the invention can be applied in absorption of acidic or basic material. For example, in case of drinking strong acid or base by mistake or excess secret of gastric juice, the high molecular weight polymer can absorb the strong acid or base to prevent tissues and cells from hazard by the strong acid or base to alleviate the uncomfortableness caused by the strong acid or base.

The high-molecular weight polymeric composition in this embodiment is mixed with a biocompatibility excipient to form a pill, a tablet, a capsule, a micro particle, a emulsion or an injection that are injected, spread, oral administrated or sprayed to the body. With reference to Fig. 1, high-molecular weight polymeric composition of the embodiment is manufactured as a pill 1 and comprises a high-molecular weight polymeric core 11 and a shell 12. The shell 12 is disposed outside of the high-molecular weight polymeric core 11 and is made of at least one biocompability biocompatibility excipient and comprises multiple holes. The toxin or the stimulating substance gets into the pill and is fixed between fibers of high-molecular weight polymeric core 11 within the high-molecular weight polymeric core 11 and is blocked to move out from the hole of the shell 12 to contact with the tissues.

Accordingly, the high-molecular weight polymeric composition promptly receives and adsorbs the toxin or stimulating substance existed in the liquid or solution to avoid the harmful toxin or stimulating substance from directly contacting to the tissue to reduce the toxicity, damage degree and side affection. The high-molecular weight polymeric composition is to directly adsorb the intaked solution or liquid to indirectly garb the harmful substance and toxin existed in the solution or liquid that is removed out of the body with the high-molecular weight polymeric composition. Therefore, the application field of the high-molecular weight polymeric composition is wider to improve the commercial potential.

### INDUSTRIAL APPLICABILITY

The invention provides a high molecular weight polymer for use in the absorption of body fluid (such as digestive juice) in an organism or a liquid or solution to be intaked into an organism containing a toxin or a stimulating component. That relates to direct absorption of a liquid or solution to indirectly adsorb the toxins or stimulating components existed therein, decrease of digestive juice in body, decrease of digestion and absorption of food in a body and improvement of excretion of these harmful substances to prevent the body from being damaged by the toxin or the stimulating substance. These high molecular weight polymers have good biocompatibility, so they will not harm health of an organism after being intaked into it. The invention applies a high molecular weight polymer to a body fluid (such as digestive juice) in an organism or a liquid or a solution to be intaked into an organism so that harmful or toxic substances contained therein can be removed through indirect adsorption or absorption and excretion. Since most high molecular weight polymers have a common feature in adsorbing liquid, they can indirectly absorb the harmful or toxic substances in the liquid. Therefore, the application field of the high-molecular weight polymeric composition is wider to improve the commercial potential.

## Claims

1. A high molecular weight polymer for use in absorbing a liquid or solution containing a toxin or a harmful substance consumed into a subject and in the removal of the toxin or the harmful substance from the subject to prevent the body from being damaged by the toxin or the stimulating substances, and
wherein the high molecular weight polymer is Sterculia BP or Frangula BPC.

2. The high molecular weight polymer for use according to Claim 1,
wherein the high molecular weight polymer is applied in the subject by injection, oral administration or spraying.

3. The high molecular weight polymer for use according to Claim 1,
wherein the toxin or harmful substance is acid, alcohol, caffeine, base, theine, or uric acid.

## Patentansprüche

1. Polymer mit hohem Molekulargewicht zur Verwendung beim Aufsaugen einer Flüssigkeit oder Lösung, die einen in eine Person aufgenommenen Giftstoff oder Schadstoff enthält, und beim Entfernen des Giftstoffs oder des Schadstoffs aus der Person, um zu verhindern, dass der Körper durch den Giftstoff oder die stimulierenden Substanzen geschädigt wird, und
wobei das Polymer mit hohem Molekulargewicht Sterculia BP oder Frangula BPC ist.

2. Polymer mit hohem Molekulargewicht zur Verwendung nach Anspruch 1,
wobei das Polymer mit hohem Molekulargewicht in der Person durch Injektion, orale Verabreichung oder Sprühen angewandt wird.

3. Polymer mit hohem Molekulargewicht zur Verwendung nach Anspruch 1,
wobei der Giftstoff oder Schadstoff eine Säure, Alkohol, Koffein, eine Base, Thein oder Harnsäure ist.

## Revendications

1. Polymère à masse moléculaire élevée pour une utilisation dans l'absorption d'un liquide ou d'une solution contenant une toxine ou une substance nocive consommée dans un sujet et dans le retrait de la toxine ou de la substance nocive du sujet pour empêcher la toxine ou les substances stimulantes d'endommager le corps, et
dans lequel le polymère à masse moléculaire élevée est Sterculia BP ou Frangula BPC.

2. Polymère à masse moléculaire élevée pour une utilisation selon la revendication 1,
dans lequel le polymère à masse moléculaire élevée est appliqué dans le sujet par injection, administration orale ou pulvérisation.

3. Polymère à masse moléculaire élevée pour une utilisation selon la revendication 1,
dans lequel la toxine ou la substance nocive est un acide, un alcool, de la caféine, une base, de la théine ou de l'acide urique.
